(19) Europäisches Patentamt
European Patent Office
Office européen des brevets

(11) Veröffentlichungsnummer: 0 144 025
A1

(12) # EUROPÄISCHE PATENTANMELDUNG

(21) Anmeldenummer: 84113801.9

(22) Anmeldetag: 15.11.84

(51) Int. Cl.⁴: C 07 D 417/12
A 61 K 31/425, A 61 K 31/43

(30) Priorität: 26.11.83 DE 3342929

(43) Veröffentlichungstag der Anmeldung:
12.06.85 Patentblatt 85/24

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Boberg, Michael, Dr.
Bergerheide 47
D-5600 Wuppertal 1(DE)

(72) Erfinder: Kinast, Günther, Dr.
Am Eckbusch 35
D-5600 Wuppertal 1(DE)

(72) Erfinder: Metzger, Karl Georg
Pahlkestrasse 75
D-5600 Wuppertal 1(DE)

(72) Erfinder: Zeiler, Hans-Joachim, Dr.
Elsbeekerstrasse 46
D-5620 Velbert 15(DE)

(54) **beta-Lactamantibiotika, Verfahren zu deren Herstellung sowie ihre Verwendung als Arzneimittel.**

(57) Die Erfindung betrifft ß-Lactamantibiotika der allgemeinen Formel I

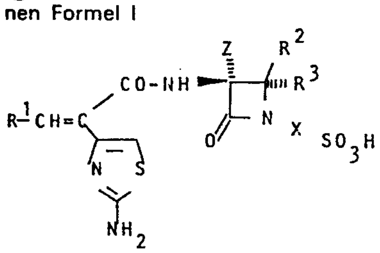

(I)

in der $R^1$, $R^2$, $R^3$, X und Z die in dier Beschreibung genannten Bedeutungen haben, Verfahren zu ihrer Herstellung sowie ihre Verwendung als Arzneimittel.

EP 0 144 025 A1

0144025

BAYER AKTIENGESELLSCHAFT 5090 Leverkusen, Bayerwerk

Konzernverwaltung RP
Patentabteilung Dn/by-c

# ß-Lactamantibiotika, Verfahren zu deren Herstellung sowie ihre Verwendung als Arzneimittel

Die Erfindung betrifft ß-Lactamverbindungen, Verfahren zu ihrer Herstellung sowie ihre Verwendung als Arzneimittel, insbesondere als antibakterielle Mittel und ferner als Mittel zur Förderung des Wachstums und zur Verbesserung der Futterverwertung bei Tieren sowie Antioxidantien.

Die Erfindung stellt neue ß-Lactamverbindungen zur Verfügung, die der allgemeinen Formel (I) entsprechen,

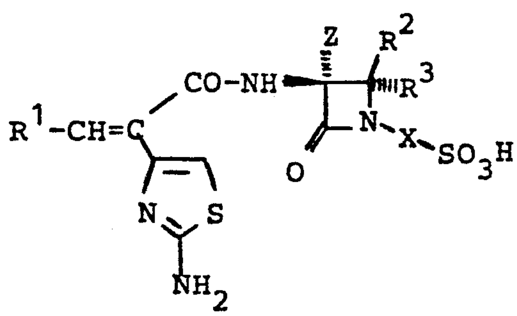

(I)

worin

X eine direkte Bindung oder Sauerstoff,

Z Wasserstoff oder Niedrigalkoxy,

$R^2$ und $R^3$ unabhängig voneinander Wasserstoff, Niedrigalkyl oder Niedrigalkoxy darstellen und

$R^1$ Wasserstoff oder gegebenenfalls substituiertes Alkyl, Aryl oder Heterocyclyl oder Hydroxycarbonyl, Niedrigalkoxycarbonyl, Halogen, Pseudohalogen oder eine Gruppe der Formel (II)

$$A-B-\overset{(O)_n}{\overset{|}{S}} \qquad (II)$$

darstellt, worin

n 0, 1 oder 2 bedeutet und

B eine direkte Bindung, Sauerstoff oder eine Gruppe $-\underset{W}{\underset{|}{N}}-$ darstellt und

A und W unabhängig voneinander Wasserstoff oder gegebenenfalls substituiertes Alkyl, Aryl oder Heterocyclyl darstellen oder gemeinsam einen gegebenenfalls substituierten carbocyclischen oder heterocyclischen Ring bilden.

In der Bedeutung von A und W steht Alkyl vorzugsweise für Niedrigalkyl und Aryl, vorzugsweise für Phenyl. Als Substituenten hierfür kommen die im folgenden für $R^1$ genannten Substituenten für Alkyl, Aryl und Heterocyclyl in Betracht.

In den Verbindungen der Formel (I) existiert zu jeder Strukturformel eine E- und eine Z-konfigurierte Verbindung gemäß der in J. Amer. Chem. Soc. 90, 509 (1968) beschriebenen E/Z-Nomenklatur.

Bevorzugte Verbindungen der Formel I liegen in der Z-Konfiguration vor.

Die Verbindungen der Formel (I) können als freie Säuren, Ester, als innere Salze oder als nicht toxische, pharmazeutisch verträgliche Salze der sauren Sulfonylgruppe, wie die Natrium-, Kalium-, Magnesium-, Calcium-, Aluminium- und Ammoniumsalze und nicht-toxische substituierte Ammoniumsalze, mit Aminen wie Di- und Triniedrigalkylaminen, Procain, Dibenzylamin, N,N'-Dibenzylethylendiamin, N-Benzyl-ß-phenylethylamin, N-Methyl- und N-Ethylmorpholin, 1-Ephenamin, Dehydroabietylamin, N,N'-Bisdehydroabiethylethylendiamin, N-Niedrigalkylpiperidin und andere Amine, die zur Bildung von Salzen von Penicillinen oder Cephalosporinen verwendet werden können, vorliegen.

Niedrigalkyl oder Niedrigalkoxy ist im Rahmen der Erfindung als Rest mit bis zu 6, vorzugsweise 1 bis 4 C-Atomen zu verstehen.

Wenn $R^1$ einen Alkylrest darstellt, dann bevorzugt einen geradkettigen oder verzweigten, gegebenenfalls substituierten Rest mit bis zu 18 C-Atomen, besonders bevorzugt mit bis zu 12 C-Atomen und speziell mit bis zu 6 C-Atomen. Alkyl im Rahmen dieser Definition umfaßt auch ungesättigte Reste vorzugsweise mit 1 oder 2 Doppelbindungen und carbocyclische Reste.

Wenn der Alkylrest $R^1$ substituiert ist, dann vorzugsweise mit einem oder zwei Substituenten, vorzugsweise aus der Gruppe Halogen, vorzugsweise F, Cl oder Br, OH, Niedrigalkoxy, Oxo, Thio, Nitro, Cyano, Hydroxycarbonyl, Niedrigalkoxycarbonyl, Aminocarbonyloxy, Sulfo, Aryl, $-O-COR^4$, $-\underset{(O)_m}{S}-R^4$ oder

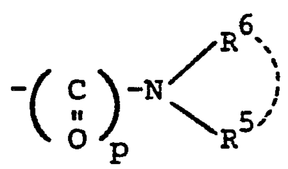

worin

m 0, 1 oder 2 bedeutet,

p 0 oder 1 bedeutet,

$R^4$ für Niedrigalkyl oder Aryl, vorzugsweise Phenyl steht und

$R^5$ und $R^6$ unabhängig voneinander Wasserstoff oder gemeinsam oder einzeln für Niedrigalkyl oder Niedrigalkanoyl stehen.

Die Bedeutung Alkyl umfaßt jeweils auch substituierte und/oder cyclische und/oder ungesättigte Strukturen.

Wenn $R^1$ einen Arylrest darstellt, dann bevorzugt einen Rest der Formel

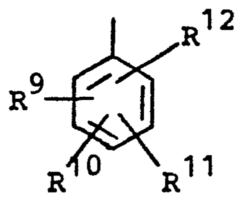

worin

$R^8$, $R^{10}$, $R^{11}$ und $R^{12}$ unabhängig voneinander Wasserstoff, Halogen, vorzugsweise F, Cl, Br, Niedrigalkyl, Aryl, eine Gruppe $-OCOR^{13}$, eine Gruppe

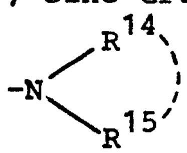 ,

Nitro, Cyano, Niedrigalkoxy, Niedrigalkylthio, Hydroxy, carbonyl, Niedrigalkoxycarbonyl, Aminocarbonyloxy, Sulfonyl oder Sulfo bedeuten,

worin wiederum Aryl einen substituierten oder unsubstituierten carbocyclischen aromatischen Ring vorzugsweise Phenyl oder einen 5- oder 6-gliedrigen heterocyclischen Ring bedeutet

und worin $R^{13}$ Niedrigalkyl bedeutet,

und worin $R^{14}$ und $R^{15}$ unabhängig voneinander Wasserstoff, oder gemeinsam oder unabhängig voneinander Niedrigalkyl oder Niedrigalkanoyl sein können.

Wenn $R^1$ einen heterocyclischen Rest darstellt, dann bevorzugt einen 5- oder 6-Ring, mit 1-4 Heteroatomen, gleich oder verschieden aus der Gruppe N, O und S. Ganz besonders bevorzugt sind die Pyridin-, Pyrimidin-, Pyrazin-, Pyridazin-, Furan-, Thiophen-, Isoxazol- oder Thiazolreste. Auch die übrigen, im Rahmen der Erfindung genannten heterocyclischen Reste sind bevorzugt die hier aufgeführten.

Bevorzugte Substituenten an einem heterocyclischen Rest dieser Definition sind Niedrigalkyl, Aryl, Halogen vorzugsweise F, Cl, Br, eine Gruppe $-OCOR^{13}$, Niedrigalkoxy, Niedrigalkylthio, Nitro, Cyano, eine Gruppe

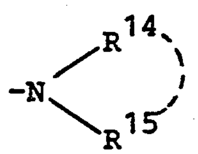, Hydroxycarbonyl, Niedrigalkoxycarbonyl,

Aminocarbonyloxy, Sulfonyl oder Sulfo,

worin Aryl wiederum einen substituierten oder unsubstituierten carbocyclischen aromatischen Ring vorzugsweise Phenyl oder einen 5- oder 6-gliedrigen heterocyclischen Ring bedeutet

und worin $R^{13}$, $R^{14}$ und $R^{15}$ die obengenannte Bedeutung besitzen.

Bevorzugte Verbindungen in der Z-Konfiguration sind in der Formel (I) solche, in denen

X eine Bindung oder Sauerstoff,

Z Wasserstoff oder Methoxy,

$R^2$ Wasserstoff,

$R^3$ Wasserstoff oder Niedrigalkyl,

$R^1$ Niedrigalkyl, einen unsubstituierten oder substituierten Phenylring, einen heterocyclischen 5- oder 6-Ring, Hydroxycarbonyl, Niedrigalkoxycarbonyl oder eine Gruppe der allgemeinen Formel (II) bedeuten,

worin

n 2,

B eine Bindung und

A Niedrigalkyl bedeutet.

Die Bedeutung Niedrigalkyl umfaßt jeweils acyclische, cyclische, gesättigte und ungesättigte Strukturen.

Die jeweiligen Z- und E-Formen derselben Strukturformel sind in der Regel unterschiedlich wirksame Substanzen, die getrennt voneinander oder gemeinsam hergestellt werden können.

Die Verbindungen der allgemeinen Formel I können dadurch erhalten werden, daß Verbindungen der allgemeinen Formel (III)

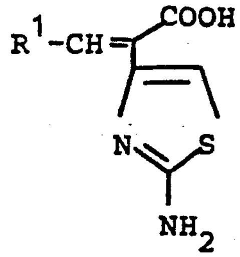

(III)

worin

$R^1$ die oben angegebene Bedeutung hat,

in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder Methansulfonsäurechlorid, nach Überführung in das Säurehalogenid oder nach Überführung in einen aktivierten Ester mit beispielsweise N-Hydroxybenztriazol und Dicyclohexylcarbodiimid, mit Verbindungen der allgemeinen Formel (IV),

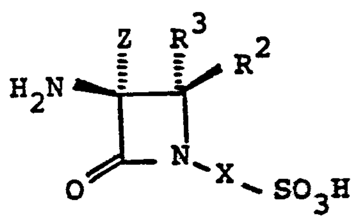

(IV)

worin

X, Z, $R^2$ und $R^3$ die obengenannte Bedeutung besitzen,

zur Umsetzung gebracht werden, dann gegebenenfalls Schutzgruppen abgespalten werden und die gewünschten Salze oder aus Salzen die freien Säuren hergestellt werden.

Für die Kupplung von Carbonsäuren (III) an ß-Lactame der Formel IV kann eine große Zahl von aus der Cephalosporin- bzw. Penicillinchemie bekannten Methoden verwendet werden. Es hat sich als vorteilhaft erwiesen, die Carbonsäuren der allgemeinen Formel III ohne Amin-Schutzgruppe zu aktivieren und dann mit den ß-Lactamen der Formel IV, die als Salze mit einem Amin in Lösung gebracht wurden, zu kuppeln. Besonders vorteilhaft ist die Aktivierung mit Sulfonsäurederivaten der Formel (V) zu Anhydriden der Formel VI

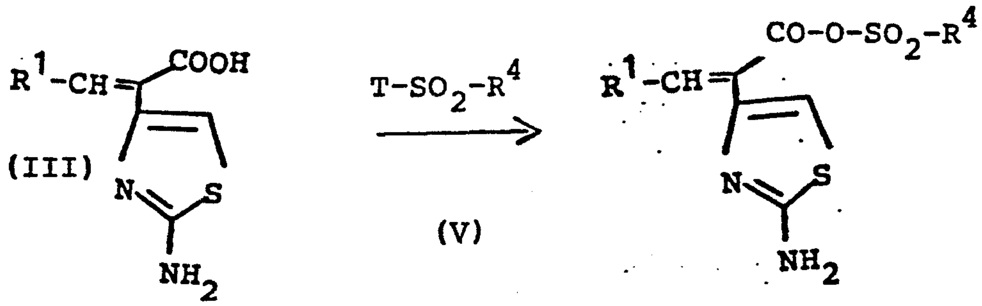

(III)

worin

T einen Rest $R^4-SO_2O$ oder Halogen darstellt und

$R^4$ einen Alkylrest mit 1-10 C-Atomen, der gegebenenfalls durch Fluor, Chlor, CN, Phenyl, Alkyloxycarbonyl, Alkyloxy oder Alkyl substituiert sein kann, wobei letztere Alkylreste 1-4 C-Atome tragen können, oder

ein Phenylrest darstellt, der gegebenenfalls durch Fluor, Chlor, Brom, CN, Alkyl, Alkyloxy, Alkylthio, Alkyloxycarbonyl - wobei letztere Alkylgruppen 1-4 C-Atome tragen können - Nitro, Trifluormethyl und Phenyl substituiert sein kann.

Wenn $R^4$ substituiert ist, sind vorzugsweise 1-3 Substituenten, vorzugsweise die genannten vorhanden.

Ganz besonders bevorzugt stellt $R^4$ einen Methyl- oder p-Tolylrest dar.

Die gemischten Anhydride der Formel VI werden hergestellt, indem man die Carbonsäuren der Formel III und 1-1,4 Äquivalente eines Amins in einem Lösungsmittel löst und mit 1 bis 1,2 Äquivalenten eines Sulfonsäurederivates der Formel V reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amin eignen sich tertiäre Amine wie z.B. Triethylamin oder Tributylamin, aber auch sterisch gehinderte sekundäre Amine wie z.B. Diisopropylamin.

Die Umsetzungen können bei Temperaturen zwischen -80°C und Raumtemperatur durchgeführt werden, wobei tiefe

Temperaturen eine Isomerisierung der Substituenten an der Doppelbindung vermeiden. Vorteilhaft wird die Aktivierung mit $Cl-SO_2CH_3$, in Dimethylformamid bei -40 bis -60°C innerhalb von 0,2 bis 24 Stunden, vorzugsweise 0,5 bis 5 Stunden, durchgeführt.

Zum Lösen der Verbindungen der Formel IV können die bei der Herstellung der Verbindungen der Formel VI genannten Lösungsmittel sowie als Base die dort genannten Amine verwendet werden.

Besonders vorteilhaft ist auch eine Aktivierung der Carbonsäuren der allgemeinen Formel III durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel VI eignen.

Die Umsetzungen können bei Temperaturen zwischen -30° und +100° durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann von ausgefallenen Dicyclohexylharnstoff abgesaugt und mit einer Verbindung der Formel IV in Form einer

Lösung ihres Aminsalzes innerhalb von 2 bis 24 Stunden umgesetzt. Zum Lösen der Verbindungen der Formel IV können die bei der Herstellung der Verbindungen der Formel VI genannten Lösungsmittel sowie als Base die dort genannten Amine verwendet werden.

Die Kupplungsprodukte der Formel I werden zweckmäßig in Form ihrer Natrium- oder Kaliumsalze direkt aus der Reaktionslösung gefällt, beispielsweise durch Zusatz von Natriumperfluorbutansulfonat und, falls erforderlich, nach Verdünnen mit einem geeigneten Lösungsmittel, vorzugsweise Aceton.

Die erfindungsgemäßen Verbindungen weisen eine starke und breite antimikrobielle Wirksamkeit besonders gegen gramnegative und gram-positive Bakterien auf. Diese Eigenschaften ermöglichen ihre Verwendung als chemotherapeutische Wirkstoffe in der Medizin. Mit ihrer Hilfe können die durch gram-negative und gram-positive Bakterien und bakterienähnliche Mikroorganismen hervorgerufenen Erkrankungen verhindert, gebessert und/ oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die

durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Gaffkya tetragena (Staph. = Staphylococcus);

Lactobacteriaceae, wie Streptokokken, z.B. Streptococcus pyogenes, α- bzw. ß-hämolysierende Streptokokken, nicht (γ-)-hämolysierende Streptokokken, Str. viridans, Str. faecalis (Enterokokken) und Dipolococcus pneumoniae (Pneumokokken) (Str. = Streptococcus);

Enterobacteriaceae, wie Escherichiae-Bakterien der Coli-Gruppe: Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marcescens (E. = Enterobaceter) (K. = Klebsiella), Proteae-Bakterien der Proteus-Gruppe: Proteus, z.B. Proteus vulgaris, Pr. morganii, Pr. rettgeri, Pr. mirabilis (Pr. = Proteus);

Pseudomonadaceae, wie Pseudomonas-Bakterien, z.B. Pseudomonas aeruginosa (Ps. = Pseudomonas);

Bacteroidaceae, wie Bacteroides-Bakterien, z.B. Bacteroides fragilis (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt:

Erkrankungen der Atemwege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calciummagnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenen Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Zur parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5,

vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 1 bis etwa 1000, vorzugsweise 1 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der

Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen ß-Lactamantibiotikum oder auch mit Aminoglykosidantibiotika, wie z.B. Gentamicin, Sisomicin, Kanamicin, Amikacin oder Tobramicin kombiniert werden.

## Beispiel 1

**(3S,Z)-3-[2-(2-Aminothiazol-4-yl)-3-pyrid-3-yl-propen-amido]-2-oxo-1-azetidinsulfonsäure-Natriumsalz**

In einem ausgeheizten Kolben wurden unter Stickstoff nacheinander 1,25 g Z-2-(2-Aminothiazol-4-yl)-3-pyrid-3-ylpropensäure, 0,5 g 1-Hydroxybenztriazol und 0,8 g Dicyclohexylcarbodiimid in 70 ml Dimethylformamid gelöst. Man rührte 5 Stunden bei Raumtemperatur, saugte dann ab und versetzte die Mutterlauge mit einer Lösung von 0,85 g (3S)-3-Amino-2-oxo-1-azetidinsulfonsäure und 0,5 ml Triethylamin in 2 ml Dimethylformamid. Nach Rühren bei Raumtemperatur über Nacht wurde die Lösung mit 1,65 g Natriumperfluorbutansulfonat versetzt, auf 2,5 ml eingeengt und dann in 200 ml Aceton eingerührt. Man saugte den Rückstand ab und trocknete im Vakuum. Man erhielt so 1,9 g der Titelverbindung.

IR (Nujol): 1765, 1655, 1615, 1530, 1270, 1245, 1200, 1050 $cm^{-1}$.

NMR (DMSO): $\delta$ = 9.22[1], J = 7Hz, 8.60[1]d, J = 3Hz, 8.49[1]dd, J = 1Hz, J = 5Hz, 7,91[1]m, 7.42[1]dd, J = 8Hz, J = 5Hz, 7.23[2]s breit, 7.20[1]s, 6,53[1]s, 4,95[1]m, 3.32[1]dd, J = 3Hz, J = 6 Hz, 3.13[1]m, ppm.

## Beispiel 2

**(3S, 4S)-3-[2-(2-Aminothiazol-4-yl)-3-methylsulfonyl-propenamido]-4-methyl-2-oxo-1-azetidinsulfonsäure-Natriumsalz**

Nach der Vorschrift von Beispiel 1 wurden 250 mg 2-(2-Aminothiazol-4-yl)-3-propensäure mit 180 mg (3S, 4S)-3-Amino-4-methyl-2-oxo-1-azetidin-sulfonsäure umgesetzt und wie folgt verarbeitet. Man versetzte mit 340 mg Natriumperfluorbutansulfonat, verdünnte mit 200 ml Aceton und 200 ml Ether und dekantierte vom ausgefallenen schmierigen Feststoff ab. Durch Verreiben mit 50 ml Methylenchlorid wurde der Feststoff langsam verfestigt und dann abgesaugt. Man erhielt 260 mg eines E/Z-Gemischs der Titelverbindung.

IR (KBr): 1745, 1611, 1521, 1126, 1039 $cm^{-1}$.

NMR ($CD_3OD$): $\delta$=7.22/7.04 [1]s, 7.14/7.02 [1]s, 4.51/4.46 [1]d, J = 3Hz, 4.27/4.15 [1]dq, J = 3Hz, J = 7Hz, 3.16/3.14 [3]s, 1.57/1.54 [3]t, J = 7Hz, ppm.

## Beispiel 3

**(3S)-3-[2-(2-Aminothiazol-4-yl)-3-methylsulfonylpropenamido]-2-oxo-1-azetidinsulfonsäure-Natriumsalz**

Nach der Vorschrift von Beispiel 1 wurde 1 g 2-(2-Aminothiazol-4-yl)-3-methylsulfonylpropensäure mit 0,7 g (3S)-3-Amino-2-oxo-1-azetidin-sulfonsäure umgesetzt. Man erhielt 1,1 g der Titelverbindung.

IR (Nujol): 1760, 1660, 1615, 1530, 1130, 1050 $cm^{-1}$.

NMR ($CD_3OD$): $\delta$= 7.15/7.03[1]s, 7.13/7.02[1]s, 5.03[1]m, 3.62-3.90[2]m, 3.14/3.10[3]s ppm.

Beispiel 4

(3S, 4S,Z)-3-[2-(2-Aminothiazol-4-yl)-3-pyrid-3-yl-propenamido]-4-methyl-2-oxo-1-azetidinsulfonsäure-Natriumsalz

Analog Beispiel 1 wurden 1,25 g Z-2-(2-Aminothiazol-4-yl)-3-pyrid-3-ylpropensäure mit 0,9 g (3S, 4S)-3-Amino-4-methyl-2-oxo-1-azetidinsulfonsäure umgesetzt. Man erhielt 1,4 g der Titelverbindung.

IR (Nujol): 1747, 1645, 1515, 1240, 1035 $cm^{-1}$.

NMR (DMSO): $\delta$= 9.25[1]d, J = 8Hz, 8.58[1]s, verbreitert, 8.45[1]dd, J = 1Hz, J = 5Hz, 8.72[1]m, 7.38[1]dd, J = 8 Hz, J = 5 Hz, 7.20[2]s breit, 7.19[1]s, 6.47[1]s, 4.48[1]dd, J = 2Hz, J = 7Hz, 3.65[1]dq, J = 2 Hz, J = 7 Hz, 1.40[3]d, J = 7 Hz, ppm.

Beispiel 5

(3S, 4S,Z)-3-/2-(2-Aminothiazol-4-yl)-3-methoxycarbonyl-propenamido7-4-methyl-2-oxo-1-azetidinsulfonsäure-Natriumsalz

0,5 g Z-2-(2-Aminothiazol-4-yl)-3-methoxycarbonylpropensäure wurden analog zu Beispiel 1 mit 0,4 g (3S, 4S)-3-Amino-4-methyl-2-oxo-1-azetidinsulfonsäure umgesetzt. Man erhielt 0,3 g der Titelverbindung.

IR (Nujol): 1760, 1665, 1530, 1250, 1055 $cm^{-1}$.

NMR ($CD_3OD$): $\delta$ = 6.90/1/s, 6.53 /1/s, 4.62/1/d, J = 2 Hz, 4.19/1/dq, J = 2 Hz, J = 7 Hz, 3.77/3/s, 1.63/3/d, J = 7 Hz, ppm.

Beispiel 6

(3S, Z)-3-/2-(2-Aminothiazol-4-yl)-3-methoxycarbonyl-propenamido7-2-oxo-1-azetidinsulfonsäure-Natriumsalz

Analog Beispiel 5 erhielt man durch Einsatz von 0,4 g (3 S)-3-Amino-2-oxo-1-azetidinsulfonsäure anstelle von (3S, 4S)-3-Amino-4-methyl-2-oxo-1-azetidinsulfonsäure 0,5 g der Titelverbindung.

IR (Nujol): 1765, 1665, 1615, 1540, 1260, 1060 $cm^{-1}$.

NMR ($CD_3OD$ + DMSO): $\delta$ = 6.92/1/s, 6.53/1/s, 5.07/1/dd, J = 6 Hz, J = 3 Hz, 3.77/3/s, 3.41/1/m, 3.20/1/m ppm.

Beispiel 7

(3S, 4S, Z)-3-/2-(2-Aminothiazol-4-yl)-2-butenamido/-4-methyl-2-oxo-1-azetidinsulfonsäure-Natriumsalz

0,55 g (3 mMol) 1-(2-Aminothiazol-4-yl)-1-propencarbonsäure in einem 1:1:1 Gemisch aus Methylenchlorid/Tetrahydrofuran/Acetonitril wurden mit 0,43 ml (3.13 mMol) Triethylamin versetzt, auf -78°C abgekühlt, dann mit 0,26 ml (3.11 mMol) Methansulfonsäurechlorid versetzt und über Nacht bei -78°C gerührt. Dann gab man 0,6 g (3.3 mMol) (3S, 4S)-3-Amino-4-methyl-2-oxo-1-azetidinsulfonsäure und 1 ml (7.15 mMol) Triethylamin in 20 ml Dimethylformamid hinzu und ließ auf Raumtemperatur aufwärmen.

Der Ansatz wurde einrotiert und der Rückstand mit 0,96 g Natriumperfluorbutansulfonat 10 Minuten verrührt. Anschließend wurden 150 ml Aceton zugesetzt und der ausgefallene Niederschlag abgesaugt und getrocknet.
Ausbeute: 0,3 g.

Beispiel 8

(3S, Z)-3-/2-(2-Aminothiazol-4-yl)-2-butenamido/-2-oxo-1-azetidinsulfonsäure-Natriumsalz

Nach der Vorschrift von Beispiel 1 wurden 0,55 g (3 mMol) 1-(2-Aminothiazol-4-yl)-1-propencarbonsäure mit 0,55 g (3.3 mMol) (3S)-3-Amino-2-oxo-1-azetidinsulfonsäure umgesetzt.
Ausbeute: 0,29 g.

Patentansprüche

1. Verbindungen der Formel (I)

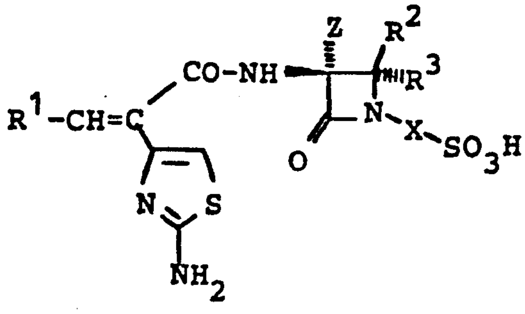

(I)

worin

X eine direkte Bindung oder Sauerstoff,

Z Wasserstoff oder Niedrigalkoxy,

$R^2$ und $R^3$ unabhängig voneinander Wasserstoff, Niedrigalkyl oder Niedrigalkoxy darstellen und

$R^1$ Wasserstoff oder gegebenenfalls substituiertes Alkyl, Aryl oder Heterocyclyl oder Hydroxycarbonyl, Niedrigalkoxycarbonyl, Halogen, Pseudohalogen oder eine Gruppe der Formel (II)

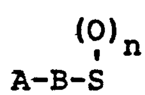

(II)

darstellt, wobei

n 0, 1 oder 2 bedeutet und

B eine direkte Bindung, Sauerstoff oder eine Gruppe -N- darstellt und
W

A und W, unabhängig voneinander Wasserstoff oder gegebenenfalls substituiertes Alkyl, Aryl oder Heterocyclyl darstellen oder gemeinsam einen gegebenenfalls substituierten carbocyclischen oder heterocyclischen Ring bilden.

2. Verbindungen nach Anspruch 1 in der Z-Konfiguration.

3. Verbindungen nach Anspruch 1 in der E-Konfiguration.

4. Verbindungen nach Anspruch 1 in denen

X eine direkte Bindung oder Sauerstoff,

Z Wasserstoff oder Methoxy,

$R^2$ Wasserstoff,

$R^3$ Wasserstoff oder Niedrigalkyl,

$R^1$ Niedrigalkyl, einen unsubstituierten oder substituierten Phenylring, einen heterocyclischen 5- oder 6-Ring, Hydroxycarbonyl, Niedrigalkoxycarbonyl oder eine Gruppe der allgemeinen Formel (II) bedeuten,

A-B-S-
$(O)_n$

worin

n 2,

B eine direkte Bindung und

A Niedrigalkyl bedeuten.

5. Pharmazeutisch verträgliche Salze und Ester der Verbindungen nach Anspruch 1 oder 2.

6. Verfahren zur Herstellung der Verbindungen nach Anspruch 1, dadurch gekennzeichnet, daß man Verbindungen der allgemeinen Formel (III)

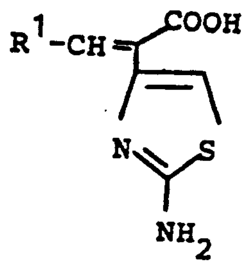

(III)

worin

$R^1$ die in Anspruch 1 angegebene Bedeutung hat,

in denen die Aminogruppe geschützt oder ungeschützt vorliegen kann, nach Aktivierung der Carboxylgruppe mit Verbindungen der allgemeinen Formel (IV)

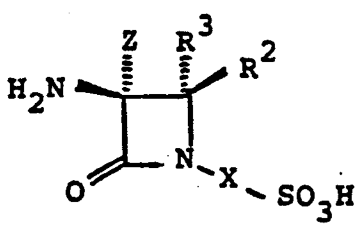

(IV)

in der

$R^2$,$R^3$,X und Z die in Anspruch 1 angegebene Bedeutung haben,

umsetzt und gegebenenfalls Schutzgruppen abspaltet und aus Salzen die freien Säuren herstellt.

7. Arzneimittel enthaltend mindestens eine Verbindung gemäß den Ansprüchen 1 bis 5.

8. Verwendung der Verbindungen gemäß den Ansprüchen 1 bis 5 bei der Bekämpfung von Krankheiten.

9. Verwendung der Verbindungen gemäß den Ansprüchen 1 bis 5 bei der Bekämpfung bakterieller Infektionen.

10. Verwendung der Verbindungen gemäß den Ansprüchen 1 bis 5 bei der Verbesserung der Futterverwertung und bei der Förderung des Wachstums bei Tieren.

Patentansprüche für Österreich

0144025

1. Verfahren zur Herstellung der Verbindungen der Formel (I)

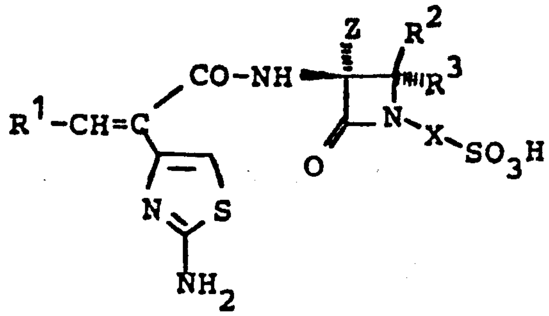

(I)

worin

X eine direkte Bindung oder Sauerstoff,

Z Wasserstoff oder Niedrigalkoxy,

$R^2$ und $R^3$ unabhängig voneinander Wasserstoff, Niedrigalkyl oder Niedrigalkoxy darstellen und

$R^1$ Wasserstoff oder gegebenenfalls substituiertes Alkyl, Aryl oder Heterocyclyl oder Hydroxycarbonyl, Niedrigalkoxycarbonyl, Halogen, Pseudohalogen oder eine Gruppe der Formel (II)

$$A\text{-}B\text{-}\overset{(O)_n}{\overset{|}{S}} \qquad (II)$$

darstellt, wobei

n 0, 1 oder 2 bedeutet und

B eine direkte Bindung, Sauerstoff oder eine Gruppe $-\underset{W}{\underset{|}{N}}-$ darstellt und

A und W, unabhängig voneinander Wasserstoff oder gegebenenfalls substituiertes Alkyl, Aryl oder Heterocyclyl darstellen oder gemeinsam einen gegebenenfalls substituierten carbocyclischen oder heterocyclischen Ring bilden,

dadurch gekennzeichnet, daß man Verbindungen der allgemeinen Formel (III)

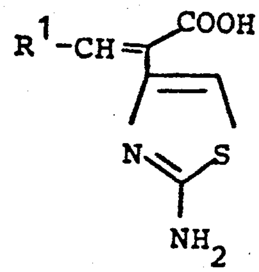

(III)

worin

$R^1$ die oben angegebene Bedeutung hat,

in denen die Aminogruppe geschützt oder ungeschützt vorliegen kann, nach Aktivierung der Carboxylgruppe mit Verbindungen der allgemeinen Formel (IV)

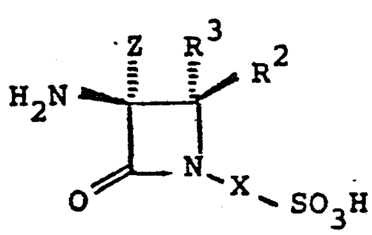

(IV)

in der

$R^2$, $R^3$, X und Z die oben angegebene Bedeutung haben,

umsetzt und gegebenenfalls Schutzgruppen abspaltet und aus Salzen die freien Säuren herstellt.

2. Verfahren nach Anspruch 1, dadurch gekennzeichnet, daß man die Carbonsäuren der allgemeinen Formel (III) ohne Amin-Schutzgruppe aktiviert und dann mit einem β-Lactam der Formel (IV), das als Salz mit einem Amin in Lösung gebracht wurde, kuppelt.

3. Verfahren nach Anspruch 2, dadurch gekennzeichnet, daß die Aktivierung mit Sulfonsäurederivaten der Formel (V)

$$T\text{-}SO_2\text{-}R^4 \qquad (V)$$

worin

T einen Rest $R^4$-$SO_2O$ oder Halogen darstellt und

$R^4$ einen Alkylrest mit 1 - 10 C-Atomen, der gegebenenfalls durch Fluor, Chlor, CN, Phenyl, Alkyloxycarbonyl, Alkoxy oder Alkyl substituiert sein kann, wobei letztere Alkylreste 1 - 4 C-Atome tragen können oder einen Phenylrest, der gegebenenfalls durch Fluor, Chlor, Brom, CN, Alkyl, Alkoxy, Alkylthio, Alkyloxycarbonyl, wobei letztere Alkylgruppen 1 - 4 C-Atome tragen können, Nitro, Trifluormethyl und Phenyl substituiert sein kann,

zu Anhydriden der Formel

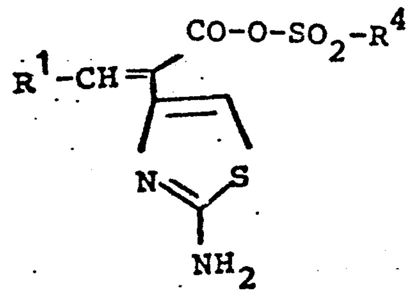

(VI)

erfolgt.

4. Verfahren nach Anspruch 3, dadurch gekennzeichnet, daß in der Verbindung der Formel (V) $R^4$ einen Methyl oder p-Tolylrest darstellt.

5. Verfahren nach Anspruch 3, dadurch gekennzeichnet, daß man die gemischten Anhydride der Formel (VI) durch Umsetzung von 1 - 1,2 Äquivalenten eines Sulfonsäurederivates der Formel (V) mit einer Lösung der Carbonsäure der Formel (III) und 1 - 1,4 Äquivalenten eines Amins in einem Lösungsmittel erhält.

6. Verfahren nach Anspruch 5, dadurch gekennzeichnet, daß man die Reaktion in Diethylether, Tetrahydrofuran, Acetonitril, Aceton, Methylenchlorid, Chloroform oder Dimethylformamid durchführt.

7. Verfahren nach Anspruch 6, dadurch gekennzeichnet, daß man die Reaktion bei Temperaturen zwischen -80°C und Raumtemperatur durchführt.

8. Verfahren nach Anspruch 1, dadurch gekennzeichnet, daß bei der Umsetzung Verbindungen der allgemeinen Formeln (III) und (IV) verwendet werden, in denen
X eine Bindung oder Sauerstoff,
Z Wasserstoff oder Methoxy,

$R^2$ Wasserstoff,

$R^3$ Wasserstoff oder Niedrigalkyl,

$R^1$ Niedrigalkyl, einen substituierten oder unsubstituierten Phenylring, einen heterocyclischen 5- oder 6-Ring, Hydroxycarbonyl, Niedrigalkoxycarbonyl oder eine Gruppe der allgemeinen Formel (II) bedeuten, worin

n 2,

B eine Bindung und

A Niedrigalkyl bedeutet.

9. Verfahren nach Anspruch 1, dadurch gekennzeichnet, daß man das Verfahrensprodukt der Formel (I) in seine Z- oder E-Formen aufspaltet.

10. Verfahren zur Herstellung von Arzneimitteln, die mindestens eine Verbindung enthalten, die gemäß den Ansprüchen 1 - 9 hergestellt worden ist.

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

0144025
Nummer der Anmeldung

EP 84113801.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 122 793 (VON HEYDEN) * Beispiele 3,6,11,12,13 * | 1-7 | C 07 D 417/12<br>A 61 K 31/425<br>A 61 K 31/43 |
| A | EP - A1 - 0 053 815 (TAKEDA) * Beispiele 102-107 * | 1-7 | |
| A | EP - A1 - 0 053 387 (TAKEDA) * Beispiele 25-27,55 * | 1-7 | |
| A | EP - A2 - 0 048 953 (E.R. SQUIBB) * Beispiele 5,6 * | 1-7 | |
| A | EP - A1 - 0 061 763 (E.R. SQUIBB) * Beispiele 5,6 * | 1-7 | |
| A | EP - A1 - 0 068 466 (E.R. SQUIBB) * Beispiel 2 * | | |

RECHERCHIERTE SACHGEBIETE (Int. Cl.4)

C 07 D 417/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7
Unvollständig recherchierte Patentansprüche: –
Nicht recherchierte Patentansprüche: 8-10
Grund für die Beschränkung der Recherche:

Art. 52(4)EPÜ; Verfahren zur therapeutischen Behandlung des menschlichen oder tierischen Körpers

| Recherchenort | Abschlußdatum der Recherche | Prüfer |
|---|---|---|
| WIEN | 11-02-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0144025

Europäisches Patentamt

# EUROPÄISCHER TEILRECHERCHENBERICHT

Nummer der Anmeldung

EP 84113801.9

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, vol. 98, no. 19, 9. Mai 1983, Columbus, Ohio, USA<br>OCHIAI, MICHIHIKO; OKADA, TAIITI; AKI, OSAMI; MORIMOTO, AKIRA; KAWAKITA, KENJI; MATSUSHITA, YOSHIHIRO "Thiazolylacetic acid compounds" Seite 513, Spalte 1, Zusammenfassung-Nr. 160 699m<br>& CA-A1-1 137 492<br>-- | 1 | |
| A | CHEMICAL ABSTRACTS, vol. 96, no. 21, 24. Mai 1982, Columbus, Ohio, USA<br>SQUIBB, E.R. AND SONS, INC. "Antibiotic β-lactams"<br>Seite 690, Spalte 2, Zusammenfassung-Nr. 181 062x<br>& Neth. Appl. NL 81 00,571<br>---- | 1 | RECHERCHIERTE SACHGEBIETE (Int. Cl.4) |